# EUROPEAN PATENT APPLICATION

(11) **EP 1 550 398 A1**
(43) Date of publication of application: **06.07.2005**
(21) Application number: 02754133.3
(22) Date of filing: 17.06.2002
(51) Int. Cl.: A61B 5/00

(54) **METHOD AND DEVICE FOR MONITORING PHYSIOLOGIC SIGNS AND IMPLEMENTING EMERGENCY DISPOSALS**

(71) Applicant: Yang, Chang-Ming, Miaoli County, Taiwan (CN)
(72) Inventor: Yang, Chang-Ming, Miaoli County, Taiwan (CN)
(74) Representative: Becker Kurig Straus
(86) International application number: PCT/CN2002/000426
(87) International publication number: WO 2003/105682

(57) **Abstract**

A method of monitoring the physiological functioning and condition of a person includes a step of using sensors in a garment body wearing by the person to continuously monitor the physiological functioning and conditions of the, and a step of using a monitoring center unit with a display for two-way inquiry or real-time treatment. The monitoring apparatus is made in the form of a LifeShirt, which is equipped with sensors and medical treating devices for monitoring the physiological functioning and conditions of the person and giving the necessary treatments, and I/O ports for communication with a remote control center.

## Description

### BACKGROUND OF THE INVENTION

### 1. Field of the Invention

The present invention relates to an apparatus for monitoring physiological functioning and condition of a person, and more particularly to the monitoring apparatus, which provides sensors and treating devices for monitoring physiological functioning and condition of a user and providing the user proper medical treatments in time when necessary and which is wearable and capable of producing signals corresponding to the physiological functioning and condition monitored.

### 2. Description of the Related Art

In addition to the monitoring index of physiological and life conditions of a person, the functioning health is also an important health index of a person.

The developing process of a disease has the so-called "incubation period". The vice president of Medical College of National Taiwan University, Professor Cheng-Yi Wang, indicated that there is a transitional period when the human body is changed from a health status to a disease status, and it is easier to cure the disease when discovered the disease before the appearance of a syndrome, i.e., at an earlier stage; certain diseases are difficult to cure even they are discovered before the appearance of a syndrome, and the patient may die quickly when a syndrome appears.

Same concept can be corresponded to the progress of aging of human beings. Aged persons faced a degeneration period when changed from healthy, active, self-managing status to a weak, sick, medical care-depending status. During such a degeneration period, many signs including lowering of vitality, increasing of time in bed, changing of living style, reducing of self-care behaviors (cooking, washing, etc.) can be observed. The concept of taking care of an old person emphasizes handling of disease suffering, malfunctioning of physiological functioning, emergency case, and other "disablement status". It is the most important and economic way to have an old person enjoy a healthy living quality by: permanently awarelessly monitoring the living environment and daily activities of the old person, establishing data transmitting, storing, handling and mining systems as well as living quality evaluating system to make a prediction upon appearance of living environment or physiological functioning deterioration signs and to inform the proper person to take the necessary measures before sick of the old person or loss of the physiological function.

Fixed detection points may be set to detect the behaviors of a person in daily life so as to collect the person's physiological data awarelessly. However, this monitoring system cannot make an accurate judgment and handling on every individual case. The monitored data can only be compared to data in record for judgment and prediction of possible progress and the way to watch.

Various physiological monitoring systems are well known. Exemplars are seen in Taiwan Patent Gazette Publication No. 383590, entitled "Wireless Physiological Condition Detector and Reporter"; Publication No. 415836 entitled "Wireless Medical Monitoring Method and Monitoring System; Publication No. 436276 entitled "Apparatus and Method for Multi-Electrode Medical Examination System". These conventional systems use devices to detect and monitor physiological conditions (body action, body temperature and pulse). When a poor physical condition occurred, fore example, excessively high or low body temperature due to external factors or poor body status, these conventional systems can only give an emergency signal to call the medical care person for help, i.e., these conventional systems provide only one-way communication and cannot actively give a treatment to maintain the user's body temperature within a proper range.

Therefore, its is desirable to provide a monitoring apparatus, which monitors the functioning of the physiological systems of the user, gives a proper treatment when necessary, and provides signal corresponding to status monitored.

### SUMMARY OF THE INVENTION

It is the primary objective of the present invention to provide a monitoring apparatus, which combines microtechnology and human factor engineering to have sensors installed in a garment to monitor the functioning of the physiological systems of the user awarelessly.

It is another objective of the present invention to provide a monitoring apparatus, which constantly monitors the functioning of the physiological systems of the user, stores, manages and analyzes monitored data for diagnosis to find out abnormal conditions, informs the medical care person to take the necessary measures when a physiological degeneration sign occurred, and gives a treatment to the user when necessary.

It is still another objective of the present invention to provide a monitoring apparatus, which is interactive with the user and capable of handling emergency conditions to take care of the user's body conditions.

To achieve these objectives of the present invention, the present invention provides a method and an apparatus for monitoring the physiological functioning and conditions of a user. The method provided by the present invention comprises the step of using sensors in a garment body wearing by a person or biochips implanted in the person to continuously monitor the physiological functioning and conditions of the person, and the step of using a monitoring center unit to transmit monitored data to a proximity or remote control center through a communication port so that the user can interact with the monitoring center unit or the user can have a two-way interaction with the remote control center, thereby providing related information to medical care persons at the remote side for diagnosis or giving an instruction to a person at the proximity side to take emergency measures.

The above-mentioned method further comprises the step of storing, managing and analyzing the monitored data for diagnosis for finding out abnormal conditions, the step of using a display to enable the user to inquire the way to treat himself or to inform the medical care person taking care of the user when a syndrome showing degeneration of the physiological functioning of the user occurred, and the step of using a video camera to pick up the images of the user and to transmit monitored images to the remote control center through the communication port, for enabling the person in charge at the remote control center to determine the necessary measures.

The apparatus provided by the present invention comprises:
a garment body which is wearable to a user and has a plurality of zones;
sensors mounted in the zones of the garment body respectively for detecting the physiological functioning and conditions of the user wearing the garment body;
medical treating devices mounted in predetermined zones of the garment body for applying medical treatments to the user wearing the garment body;
a communication port for transmitting the monitored data to a remote control center on the real time or at a delayed time or receiving and answering the inquiries of the user, the communication port being electrically connected to the medical treating devices;
a monitoring center unit electrically connected with the sensors, the medical treating devices and the communication port for receiving and transmitting signals such that the communication port is used to transmitting the monitored data to the remote control center, the monitoring center having I/O ports connectable to the sensors and the medical treating devices;
whereby the monitoring data of the user's physiological functioning and conditions is stored, managed and analyzed to find out abnormal conditions of the user for further treatments.

Wherein the sensors are selected from the group consisting of pressure sensors, temperature sensors, terminal sensors, voice sensors, biochemical sensors and biochips.

Wherein the sensors produce signals corresponding to the physiological functioning and conditions of the user and send the signals to the communication port.

Wherein the medical treating devices are selected from the group consisting of oxygen source devices, pumps, air bags, body temperature regulators, pain-causing devices, hypodermic syringes and electroshock devices.

The air bag is used with the pump, the oxygen source device or the sensors to correct the posture of the user, to fix a broken bone in position, to impart a pressure to the user, to stop bleeding of blood, to apply cardio-pulmonary resuscitation or abdominal thrust (Heimlich maneuver) to the user.

The air bag is supported on a bracket at the garment body for supporting the spine of the user wearing the garment body in shape.

Wherein the communication port is connectable with a communication device to transmit monitored data to the remote control center for remote diagnosis, a computer or other compatible devices.

Wherein the monitoring center unit further comprises:
a sensor interface electrically connected to the sensors to transmit detected data to a processor for computing;
a communication port for transmitting detected data to the remote control center through a communication device for remote diagnosis, or to a computer or other compatible devices;
a data storage device for storing input data and detected data;
a display disposed at the garment body for displaying information; and
a power system for providing the apparatus with the necessary working electricity.

The apparatus further comprises means for data searching for enabling the monitoring center unit to be set for individual use subject to personal data inputted therein.

### BRIEF DESCRIPTION OF THE DRAWINGS

FIG. 1A is a front view of a monitoring apparatus according to a preferred embodiment of the present invention.
FIG. 1B is a rear view of the monitoring apparatus shown in FIG. 1A.
FIG. 2 is a circuit block diagram of the electroshock device according to the preferred embodiment of the present invention.
FIG. 3 is a schematic sectional view in an enlarged scale of a part in FIG. 1B, showing a narrow elongated air bag extending in vertical direction corresponding to the user's spine and a bracket supporting the narrow elongated air bag.
FIG. 4 is a system block diagram of the monitoring center unit according to the preferred embodiment of the present invention.
FIGS. 5A-5C are operation flow charts of the preferred embodiment of the present invention.

### DETAILED DESCRIPTION OF THE INVENTION

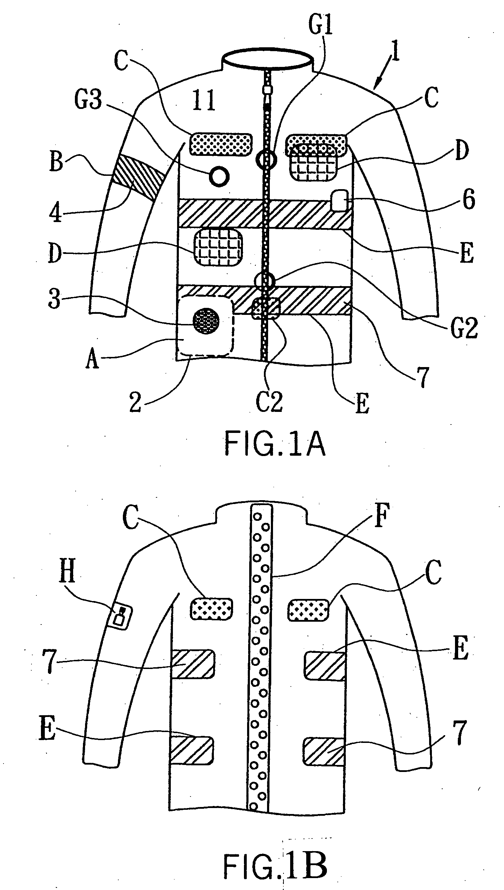

FIG. 1A shows a monitoring apparatus **1** for monitoring the physiological functioning and conditions of a human being according to a preferred embodiment of the present invention. The monitoring apparatus **1** may be made in any of a variety of forms.

According to this embodiment, the monitoring apparatus **1** is made in the form of a jacketing, comprising a garment body **11.** The garment body **11** is equipped with a plurality of sensors and medical treating devices, a communication port **232** for transmission of processing data, and a plurality of I/O ports connectable to external information equipments. Unlike short-time inquiries test and diagnosis in hospital, the sensors and medical treating devices at the garment body **11** are practical for long-term and continuous monitoring of the functioning of physiological systems of a person.

The garment body **11** is divided into multiple zones (**Zones A-H**) and the sensors and/or medical treating devices may be installed in the outer side or inner side of the garment body **11** at these zones.

**Zone A** comprises a monitoring center unit **2.** The monitoring center unit **2** comprises a communication port **232,** an integrated circuit (IC), and an oxygen can or mini air pump **3** adapted to prevent an abrupt condition due to malfunctioning of the user's breathing system. The mini air pump **3** can also be used to provide the necessary air to inflate other devices under an emergency condition. The communication port **232** has terminals connectable to a communication device, for example, cell phone, global positioning system (GPS), radio, personal digital assistant (PDA) for transmitting or receiving data. When the user is in an emergency case, the user or a third party can use the attached communication device to communicate with the clinicians at the remote site in charge of the monitoring job, asking for help. Through the GPS, the clinicians at the remote side can learn the location of the user of the monitoring apparatus **1.**

**Zone B** is at the upper arm of the monitoring apparatus **1,** comprising an annular cuff **4** with a pressure sensor (not shown) electrically connected to the monitoring center unit **2** and mechanically connected to the mini air pump **3** for measuring the user's blood pressure. In the same way, a thermometer that is installed in other zones can be used to measure the user's body temperature. Similarly, other sensors may be used to measure the pulse beat, blood oxygen concentration, blood glucose, and other fundamental physiological conditions of the user.

If the user's body temperature goes higher than normal due to environment causes or illness, a gas cylinder (not shown) in the garment body **11** is opened to discharge a gas to lower the user's body temperature. On the contrary, if the user's body temperature goes lower than normal, a heater powered by external electricity is started to keep the user's body warm.

**Zone C** comprises a voice sensor (not shown) adapted to detect abnormal conditions of the heart and the lungs. The voice sensor can be a piezoelectric or capacitive microphone. **Zone C2** comprises a voice sensor for fetal monitoring. The voice sensors in **Zone C** and **Zone C2** are respectively electrically connected to the monitoring center unit **2.**

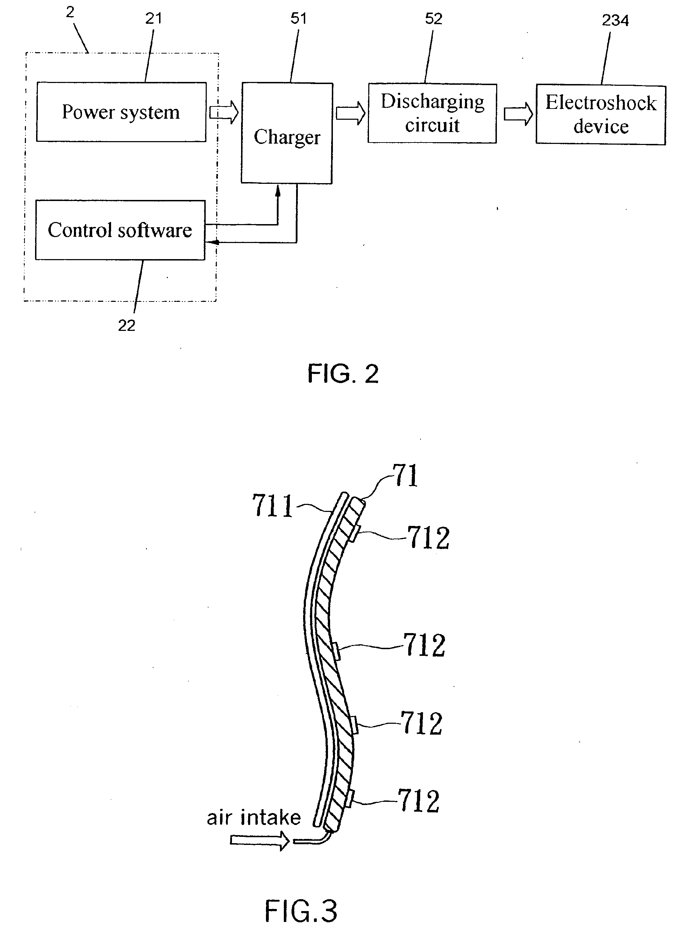

**Zone D** comprises a charging discharging circuit with an electroshock device **234.** Referring also to FIG. 2, the monitoring center unit **2** further comprises a power system **21** adapted to convert electric energy into the desired voltage level, a charger **51** adapted to store electric energy obtained from the power system **21,** a discharging circuit **52** adapted to discharge electricity from the charger **51** through the electroshock device **234,** and a control software **22** adapted to control the discharging amount and time of electricity from the charger **51** to the discharging circuit **52,** and a cardioscope monitor sensor **6** adapted to detect the user's heart beat and to output the monitoring result (cardiograph) through the monitoring center unit **2. Zone D** further comprises a plurality of air bags **G1-G3** mounted inside the garment body **11.** Air bag **G1** is inflatable and debatable by the mini air pump **3** by means of the control of the control software **22,** and adapted to apply massage to the user when a cardio-pulmonary resuscitation is practiced. Air bag **G2** is adapted to apply an abdominal thrust (Heimlich maneuver) to the user. Air bag **G3** is adapted to give a sudden pressure to stimulate the user to determine whether the user is still conscious and aware when the user is in comma.

**Zone E** comprises an air bag **7** partially extending to or placed at the user's back for correcting the user's posture. Alternatively, two air bags may be installed and alternatively inflated to turn the user' back between two positions.

**Zone F** (see FIG. 1B) comprises a narrow elongated air bag **71** extending in vertical direction corresponding to the user's spine (see FIG. 3), and a bracket **711** mounted in the garment body **11** and adapted to support the air bag **71** in shape. The air bag **71** is inflatable by the mini air pump **3,** and adapted to correct the posture of the user's spine and to support the user's spine in position. Further, sensors **712** are installed in the air bag **71** and kept in contact with the user's back, waist and hip areas to detect the posture of the user's spine and to provide detected data to the monitoring center unit **2** for enabling the monitoring center unit **2** to control inflation of the air bag **71.**

**Zone H** comprises a hypodermic syringe **235** controlled by the person at the remote site in charge of the monitoring job through the monitoring center unit **2** via the mini air pump **3** to inject medicine prepared subject to the doctor's prescription into the user's body when necessary.

The number of zones and related sensors and medical treating devices may be adjusted subject to the user's conditions to achieve the best performance: For example, after having the monitoring apparatus **1** put on the user, we can real-time monitor the user's physiological conditions such as heart beat, pulse, breathing status, body temperature, glucose, and blood pressure. When the monitoring center unit finds that any one of the parameters goes beyond normal for a while without improvement, the monitoring center unit immediately sends a signal to inform the user's family or the person taking care of the user, and the user can find out how to treat this situation through a display **24.** If the user's family or the user cannot handle the case, the images or an emergency call signal can be sent through the communication port **232** to the person at the remote side in charge of the monitoring job via a cell phone or PDA. The person at the remote side in charge of the monitoring job can then decide to take the necessary steps subject to the provided data. The person at the remote side in charge of the monitoring job can also provide monitoring data to the doctor for reference so that the doctor can make a proper evaluation. When detected the sign that shows degeneration of the user's physiological systems, the user's family or the person taking care of the user immediately know the situation and can take the necessary measures to improve the situation. Further, it is necessary to receive training before use of the monitoring apparatus.

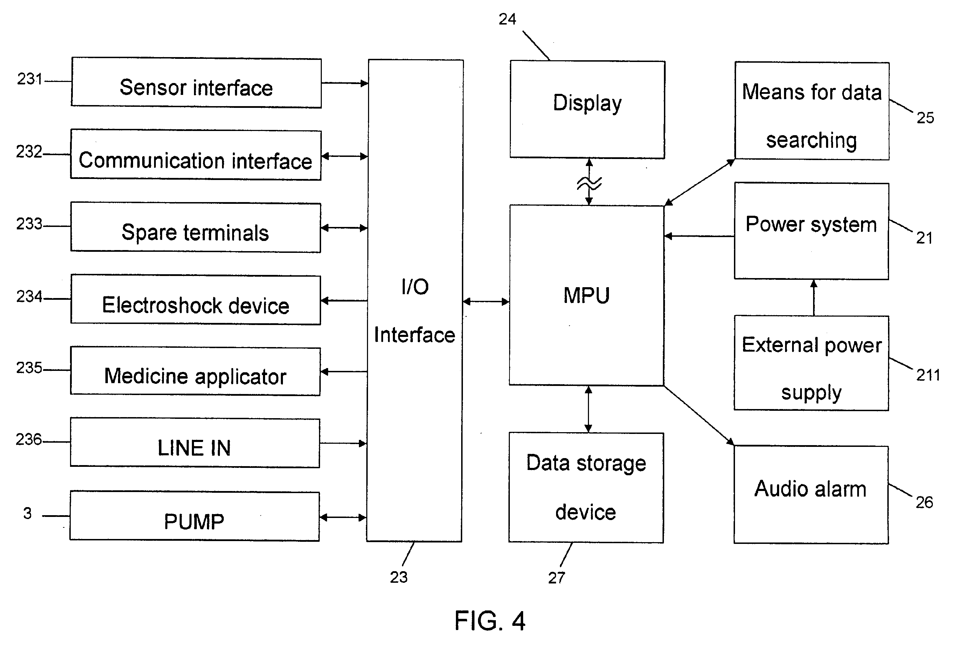

FIG. 4 is a monitoring center unit system block diagram of the present invention. The system comprises a number of I/O interfaces **23,** a sensor interface **231** having electrically connected thereto the sensors in the garment body of the monitoring apparatus for transmitting detected data to the main processing unit for processing, a communication interface **232** for sending detected data to the remote side through a communication device or directly to a computer or other apparatus, and for remote diagnosis from the remote side, a spare terminals **233** for the connection of other medical devices, a data storage device **27** for storing data obtained from the sensors in the garment body, a display **24** provided outside the garment body for data output, a power system **21** adapted to provide the whole system with the necessary working voltage, an electroshock device **234,** which obtains the necessary working voltage from an external power supply **211** through the power system **21,** an audio alarm **26** adapted to provide an audio warning signal at the set time to take medicine or when an abrupt physical condition of the user occurs.

The system further has means for data searching **25** connected thereto for enabling the monitoring center unit to search monitored data of set items. For example, if it needs only to monitor the user's the heart or breathing system, a pre-set data chip is inserted into the system, and the system will turn off other sensors and medical treating devices to save power consumption. By means of an external control, the user can change the settings and add new monitoring items.

Everyone can be a medical care person capable of using the monitoring apparatus of the invention. This monitoring apparatus is practical for use in a remote place lacking in medical facilities, or for a person to be constantly monitored. The user can communicate with the monitoring system or the remote control center to provide related data to the medical care person at the remote control center so that the medical care person at the remote control center can take the necessary measures when necessary.

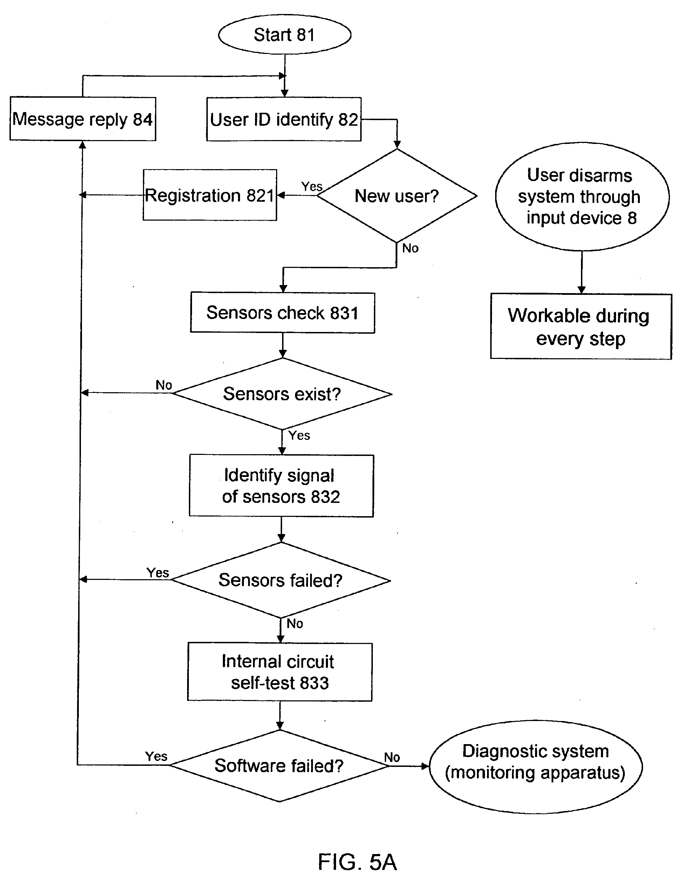

FIG. 5A is a monitoring and diagnostic flow of the present invention. When started (step **81**), it recognizes the user's identification (step **82**) and requests registration of person data (step **821**) in case of a new user. After recognition of the user's ID, the diagnostic system (the monitoring apparatus) runs self-test on every device (steps **831-833**) to check if sensors exist or not (step **831**), if sensors functioning normal or not (step **832**), if software functioning normal or not (step **833**). When all checked OK, the diagnostic system enters stand-by mode. When an abnormality occurred during self-test, the diagnostic system immediately gives a respective message (step **84**) through the display. The user can input an instruction to disarm the system when desired during running of the system (step **8**).

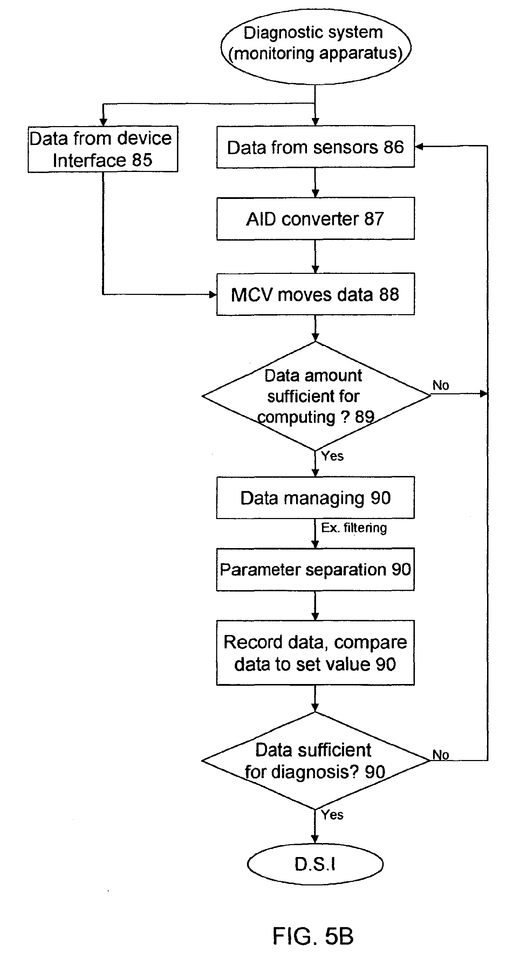

Referring to FIG. 5B, we have the data to be obtained through two ways, i.e., from the apparatus interface (step **85**) or through the respective sensor (step **86**) (for example, body temperature). The data obtained from the sensor must be converted through an A/D converter (step **87**) into MCU moves data (step **88**). At this time, MCU judges if the provided data reaches the least computing unit (step **89**). If provided data is insufficient, MCU keeps requesting further data. When sufficient data obtained, the obtained data is compared to the pre-set reference value (step **90**) (for example, the reference value is set to be 37°C for body temperature), and an examination result is reported subject to the comparison result.

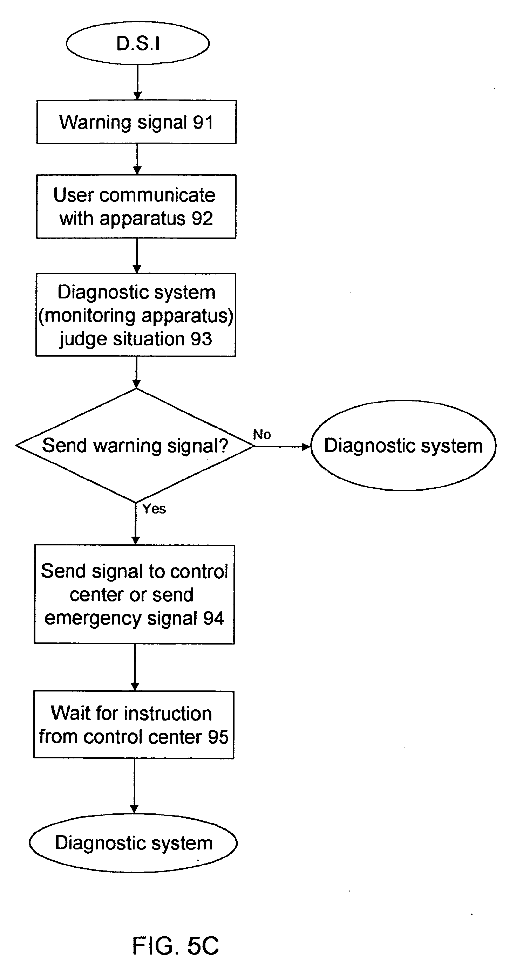

Referring to FIG. 5C, after power on and self-test, the monitoring apparatus of the present invention controls the respective sensors to monitor the functioning of the physiological systems of the user. When an abnormal condition occurred (step **91**) (for example, body temperature over 39°C), the user can immediately communicate with the diagnostic system through an input device (step **92**) by means of a question and answer mode (for example, the apparatus suggests the user to apply cryotherapy or to start the cooling device). If the user's body temperature is not improved significantly and the user has another syndrome (for example, headache), the MCU judges the situation subject to information inputted by the user (step **93**) (evaluate the functioning of other physiological systems). The inputted information is sent to the diagnostic system through the I/O interface so that the diagnostic system can suggest giving oral antipyretic or painkiller. When necessary, the MCU gives an emergency call signal through the I/O interface to the medical care center (step **94**), so that the doctor can give instruction to send the user to hospital or to take necessary measures (step **95**). When waiting for rescue, the monitoring apparatus provides the user with certain measures such as cardio-pulmonary resuscitation, body temperature maintaining, emergency medicine application, electroshock application, etc.

## Claims

1. A method of monitoring the physiological functioning and conditions of a person comprising the step of using sensors in a garment body wearing by the person or biochips implanted in the person to continuously monitor the physiological functioning and conditions of the person, and the step of using a monitoring center unit to transmit monitored data to a proximity or remote control center through a communication port so that the user can interact with the monitoring center unit or the user can have a two-way interaction with the remote control center, thereby providing related information to medical care persons at the remote side for diagnosis or giving an instruction to a person at the proximity side to take emergency measures.

2. The method as claimed in claim 1, further comprising the step of storing, managing and analyzing the monitored data for diagnosis for finding out abnormal conditions, the step of using a display to enable the user to inquire the way to treat himself or to inform the medical care person taking care of the user when a syndrome showing degeneration of the physiological functioning of the user occurred, and the step of using a video camera to pick up the images of the user and to transmit monitored images to the remote control center through the communication port, for enabling the person in charge at the remote control center to determine the necessary measures.

3. An apparatus for monitoring the physiological functioning and conditions of a user, comprising:
a garment body wearable to a user, the garment body having a plurality of zones;
sensors mounted in the zones of the garment body respectively for detecting the physiological functioning and conditions of the user wearing the garment body;
medical treating devices mounted in predetermined zones of the garment body for applying medical treatments to the user wearing the garment body;
a communication port for transmitting the monitored data to a remote control center on the real time or at a delayed time or receiving and answering the inquiries of the user, the communication port being electrically connected to the medical treating devices;
a monitoring center unit electrically connected with the sensors, the medical treating devices and the communication port for receiving and transmitting signals such that the communication port is used to transmitting the monitored data to the remote control center, the monitoring center having I/O ports connectable to the sensors and the medical treating devices;
whereby the monitoring data of the user's physiological functioning and conditions is stored, managed and analyzed to find out abnormal conditions of the user for further treatments.

4. The apparatus as claimed in claim 3, wherein the sensors are selected from the group consisting of pressure sensors, temperature sensors, terminal sensors, voice sensors, biochemical sensors and biochips.

5. The apparatus as claimed in claim 3 or claim 4, wherein the sensors produce signals corresponding to the physiological functioning and conditions of the user and send the signals to the communication port.

6. The apparatus as claimed in claim 3, wherein the medical treating devices are selected from the group consisting of oxygen source devices, pumps, air bags, body temperature regulators, pain-causing devices, hypodermic syringes and electroshock devices.

7. The apparatus as claimed in claim 6, wherein the air bag is used with the pump, the oxygen source device or the sensors to correct the posture of the user, to fix a broken bone in position, to impart a pressure to the user, to stop bleeding of blood, to apply cardio-pulmonary resuscitation or abdominal thrust (Heimlich maneuver) to the user.

8. The apparatus as claimed in claim 7, wherein the air bag is supported on a bracket at the garment body for supporting the spine of the user wearing the garment body in shape.

9. The apparatus as claimed in claim 3, wherein the communication port is connectable with a communication device to transmit monitored data to the remote control center for remote diagnosis, a computer or other compatible devices.

10. The apparatus as claimed in claim 3, wherein said monitoring center unit further comprises:
a sensor interface electrically connected to the sensors to transmit detected data to a processor for computing;
a communication port for transmitting detected data to the remote control center through a communication device for remote diagnosis, or to a computer or other compatible devices;
a data storage device for storing input data and detected data;
a display disposed at the garment body for displaying information; and
a power system for providing the apparatus with the necessary working electricity.

11. The apparatus as claimed in claim 3 or claim 10, further comprising means for data searching for enabling the monitoring center unit to be set for individual use subject to personal data inputted therein.
